# EUROPÄISCHE PATENTSCHRIFT

(11) **EP 1 179 325 B1**
(45) Veröffentlichungstag und Bekanntmachung des Hinweises auf die Patenterteilung: **01.06.2005**
(21) Anmeldenummer: 01117921.5
(22) Anmeldetag: 24.07.2001
(51) Int. Cl.: A61F 5/01, A61F 13/06

(54) **Bandage für das Sprunggelenk**
Ankle joint bandage
Bandage pour la cheville

(30) Priorität: 29.07.2000 DE 10037342
(43) Veröffentlichungstag der Anmeldung: 13.02.2002
(73) Patentinhaber: Beiersdorf AG, 20253 Hamburg (DE)
(72) Erfinder: Herzberg, Thorsten, 22419 Hamburg (DE)

(56) Entgegenhaltungen:
- EP-A- 0 071 212
- EP-A- 0 931 528
- DE-A- 3 840 714
- DE-A- 3 909 922
- DE-C- 503 167
- DE-C- 4 291 109
- DE-C- 4 318 588
- GB-A- 539 243
- US-A- 4 085 746
- US-A- 4 367 733

## Beschreibung

Die Erfindung beschreibt eine Bandage für Verletzungen des Sprunggelenkes, zum Beispiel Distorsionen, Torsionen der leichteren Art, Dehnungen im fibularen Bereich so wie der lateralen Bandstruktur und beginnenden degenerativen Veränderungen des oberen und/oder unteren Sprunggelenkes.

Orthopädische Bandagen üben entsprechend ihrer Konstruktion und ihrem Indikationsfeld eine fixierende, führende, stützende und/oder unterstützende Funktion auf die Extremitäten des menschlichen Körpers aus.
Diese medizinischen Bandagen müssen eine Form aufweisen, um den anatomischen Gegebenheiten zu entsprechen, um form- und kraftschlüssig von extern auf den menschlichen Körper einwirken zu können.

Die Herstellung von solchen medizinischen Bandagen erfolgt durch Ausschneiden von Zuschnitten aus flächigem Material, zum Beispiel aus Neopren, Gewirke, oder Geweben. Die anatomiegerechte Form wird durch die Form der Zuschnitte oder Abnäher, zum Beispiel mit Zwickeln, und das anschließende Zusammenfügen der Zuschnitte erreicht, so wie es auch bei Bekleidung üblich ist.

Das Zusammenfügen kann durch Nähen, Kleben oder andere übliche Verfahren erfolgen. Der große Nachteil dieser Bandagen ist, daß die genaue, anatomische Paßform nur schwierig erreicht werden kann und eine Vielzahl von Verbindungsstellen entstehen, beispielsweise Nähte. Diese Verbindungsstellen verändern die Eigenschaften des eingesetzten Materials, und es besteht die Gefahr von Druckstellen auf der Haut.

Sprunggelenkorthesen oder -bandagen dienen bevorzugt für die frühfunktionelle Behandlung frischer fibularer Bandrupturen und von leichten und mittelschweren Fußwurzeldistorsionen, als auch für die Anwendung bei chronischer Instabilität.

Durch die DE-A-38 40 714 ist eine Sprunggelenkorthese mit einem U-förmigen Stützbügel bekannt, dessen Schenkel unterhalb des Fußes in einem Steg zusammenlaufen, über die Knöchel hinaufreichen und in ihrem Endbereich durch ein Befestigungsband zusammen gehalten sind. Dabei ist der äußere Schenkel seitlich vor seinem Knöchel und der innere Schenkel in Gegenüberstellung zum äußeren Schenkel vor der Achillessehne hochgeführt. Beide Schenkel sind in Richtung zu dem Steg bis zu einer Lage vor der Ferse geführt und verlaufen in Richtung zu ihren Enden aufwärts derart, daß sie seitlich neben den Schienbeinkanten etwa parallel zu diesen aufwärts streben, wobei im unteren Bereich der Schenkel ein Halteband angebracht ist, das von dem einen Schenkel über den Rist schräg aufwärts zum anderen Schenkel an diesem festlegbar verläuft, oberhalb der Knöchel um die Achillessehne greift und auf dem Rist sich überkreuzend an dem anderen Schenkel in einem Halteteil endet. Mit einer derart ausgebildeten Sprunggelenkorthese soll das Umknicken vor allem in Richtung seitvom, also in Richtung auf eine Spitzfuß-Stellung, verhindert werden. Da der U-förmige Stützbügel dieser Sprunggelenkorthese mit seinem äußeren Schenkel seitlich vor dem Fußknöchel und mit seinem inneren Schenkel vor der Achillessehne hochgeführt und von einem unterhalb des Fußes verlaufenden Steg zusammengehalten sind, wird der mediale Rand des Mittelfußes nicht erfaßt, so daß der Einsatz dieser Sprunggelenkorthese begrenzt ist.

Eine Fußfixierungsschiene beschreibt die DE-A-39 09 922. Diese Fußfixierungsschiene dient insbesondere zur postoperativen Behandlung eines verletzten Sprunggelenkes mit einem den Fuß umfassenden Fußteil, an den sich nach oben bis in den Wadenbereich reichend ein mit Verschlußzügeln versehener Halteteil anschließt. Der Halteteil ist dabei in zwei Seitenteile unterteilt, die an den Fußteil anschließen und schalenförmig ausgebildet sind. Jeweils der den Fußknöchel überdeckende Bereich des Seitenteils ist mit einer fensterartigen Ausnehmung versehen. Der Bereich der Achillessehne am Fußteil und am Halteteil ist dabei ausgespart. Die einstell- und feststellbaren bandförmigen Verschlußzügel bestehen aus einem undehnbaren Material, wobei ein Verschlußzügel an dem Fußteil so angeordnet ist, daß er den Fußrücken übergreifend den ersten Strahl des Mittelfußes gegen supinatorisches Aufsteigen fixiert. Mit einer derartigen Fußfixierungsschiene soll zum einen eine einwandfreie Ruhigstellung des zu behandelnden Fußes erreicht werden, und zum anderen sollen die Nachteile eines Gipsverbandes vermieden werden, denn nach Verletzungen und Operationen am äußeren Bandapparat wird oftmals der Fuß zur Ruhigstellung eingegipst, wobei eine postoperative Behandlung von Operationswunden wegen zahlreicher gravierender Nachteile nicht möglich ist. Bei dieser Fußfixierungsschiene wird von einer U-förmigen Gelenkmanschette mit vollflächigem Sohlenteil ausgegangen, das den Mittel- und Vorfuß bis zum kleinen Zehenballen erfaßt, wobei jedoch keine ausreichende Gelenkigkeit im Mittelfußbereich gegeben ist.

Die DE-C-43 18 588 offenbart ebenfalls eine Sprunggelenkorthese, die aus einer U-förmigen, aus thermoplatischem Material hergestellten Gelenkmanschette besteht, die sich aus einer Außenknöchelschiene und einer Innenknöchelschiene zusammensetzt. Diese Knöchelschienen sind durch einen unter der Ferse verlaufenden Steg verbunden. Die Knöchelschienen weisen ferner anatomisch gerechte Vertiefungen zur Anpassung an die Knöchelkonturen und zum anatomiegerechten Sitz auf. Ein weiteres Element der Orthese ist sein Mittelfußteil, das ebenfalls aus thermoplastischem Material gefertigt ist. Dieser Teil der Orthese läuft quer unter der Fußsohle proximal der Köpfchen der Mittelfußknochen I-V und ist medial und lateral laschenförmig ausgebildet. Die so ausgebildeten Laschen umfassen die Fußränder außen und innen. Sie führen zum einen den Mittelfuß, zum anderen dienen sie zur Befestigung von Kreuz- und Quergurtbändem. Das Mittelfußteil ist dabei fußsohlenseitig durch einen ebenfalls aus thermoplastischem Material hergestellten, jedoch hochflexiblen Steg verbunden. Dieser Steg hat die Funktion eines Gelenkes und arbeitet analog einem Filmschamier. Die Drehachse dieses in dem hochflexiblen Steg ausgebildeten Gelenkes verläuft von dorso-medial nach antero-lateral und bildet mit der Fußlängsachse einen Winkel von etwa 10°, und zwar entsprechend der Anatomie des unteren Sprunggelenks.

Die DE 42 91 109 C2 offenbart eine Stützvorrichtung für ein Fußgelenk mit einem verschließbaren Hauptstützkörper, der ein Unterschenkelteil und ein Fußteil aufweist, sowie mit einem inneren und/oder äußeren Stützteil, die jeweils an einem Seitenteil des Hauptstützkörpers angebracht sind, wobei der Unterschenkelteil und der Fußteil senkrecht zueinander stehen. Die Stützteile sind abnehmbar am Hauptstützkörper angebracht.
Sowohl das Unterschenkelteil und als auch das Fußteil verfügen über Klettverschlüsse, die zum Anlegen der Stützvorrichtung verschlossen werden können.
Die Stützteile weisen darüber hinaus an ihrem Ende in Richtung der Fußsohle die Möglichkeit auf, diese über ein Koppelband, das unter der Fußsohle verläuft, zu verbinden.

Schließlich ist an dem Fußteil mindestens ein Dehnungsband vorhanden, das mit einem Ende an der Außenseite des Fußsohlenbereichs befestigt ist und vom Fußsohlenbereich über den Fußrückenbereich hinweg geführt werden kann, um spiralförmig um den Unterschenkelbereich geschlungen zu werden.

Der Erfindung liegt die Aufgabe zugrunde, eine Bandage zu konzipieren, die ein hohes Maß an Funktionalität mit propriozeptiver Wirkung aufweist, gleichzeitig aber einfach und unkompliziert für den Patienten anzulegen ist und durch den Verzicht von starren Elementen ein hohes Maß an Dynamik aufweist. Außerdem soll die Bandage kostengünstig herzustellen sein.

Diese Aufgabe wird durch die gemäß Hauptanspruch gekennzeichnete Bandage gelöst. Gegenstand der Unteransprüche sind dabei vorteilhafte Weiterbildungen der Bandage.

Demgemäß betrifft die Erfindung eine Bandage für das Sprunggelenk mit einem Unterschenkelteil und mit einem Fußteil mit zwei Zügeln.
Das Unterschenkelteil weist zumindest einen Zügel auf, das Fußteil an der medialen Seite einen ersten Zügel und an der lateralen Seite einen zweiten Zügel.
Das Unterschenkelteil und das Fußteil sind erfindungsgemäß so miteinander verbunden, insbesondere vernäht, daß bei am Fuß angelegter Bandage die Fersenpartie des Fußes ausgespart wird.

Bei angelegter Bandage umfaßt das Unterschenkelteil im distalen Unterschenkelbereich den Unterschenkel posterior zirkulär und ist verschließbar, insbesondere anterior. Das Fußteil liegt unter der Fußsohle und umfaßt den Vorfuß mit dem medialen und dem lateralen Zügel, wobei die Zügel, vom plantaren Bereich kommend, sich kreuzend über dem Fußrücken nach medial und lateral zum distalen Bereich des Unterschenkels verlaufen.

In einer ersten bevorzugten Ausführungsform der Bandage ist ein Verbindungsteil mit dem Unterschenkelteil und dem Fußteil verbunden, ebenfalls bevorzugt vernäht, wobei das Verbindungsteil bei angelegter Bandage im distalen Unterschenkelbereich den Unterschenkel anterior und den Vorfuß dorsal umfaßt.

Das Verbindungsteil versetzt die Bandage in einen schlauchförmigen Zustand, so daß im anterioren Bereich des Unterschenkels sowie im dorsalen Bereich des Fußes ein sicheres Anlegen und Positionieren der Bandage erreicht und somit die Therapiesicherheit erhöht wird.

Weiter vorzugsweise weist das Unterschenkelteil einen medialen Zügel und einen lateralen Zügel auf, die den Unterschenkel posterior zirkulär umfassen und anterior verschlossen werden.

In einer weiteren bevorzugten Ausführungsform sind das Unterschenkelteil und das Fußteil der Bandage aus einem mindestens beidseitig kaschierten Material gefertigt, das ein klettfähiges Velour zur Außenseite und ein hautfreundliches textiles Gewebe zur Innenseite aufweist.
Ebenfalls können Abstandsgewebe mit entsprechender Kaschierung oder im Falle einer technischen, maschinellen Ausrüstung auch ohne Kaschierung verwendet werden.
Derartige Abstandsgewebe werden in der EP 0 071 212 B1 offenbart. Abstandsgewebe sind mattenförmige Schichtkörper mit einer Deckschicht aus einem Faser- oder Filamentvlies, einer Unterlagsschicht und zwischen diesen Schichten vorhandene einzelne oder Büschel von Haltefasern, die über die Fläche des Schichtkörpers verteilt durch die Partikelschicht hindurchgenadelt sind und die Deckschicht und die Unterlagsschicht untereinander verbinden. Als zusätzliches, aber nicht erforderliches Merkmal sind gemäß EP 0 071 212 B1 in den Haltefasem Partikel aus inerten Gesteinspartikeln, wie zum Beispiel Sand, Kies oder dergleichen, vorhanden.
Die durch die Partikelschicht hindurchgenadelten Haltefasern halten die Deckschicht und die Unterlagsschicht in einem Abstand voneinander, und sie sind mit der Deckschicht und der Unterlagsschicht verbunden.
Abstandsgewebe oder -gewirke sind u. a. in zwei Artikeln beschrieben, und zwar
einem Artikel aus der Fachzeitschrift "kettenwirk-praxis 3/93", 1993, Seiten 59 bis 63 "Raschelgewirkte Abstandsgewirke"
und
einem Artikel aus der Fachzeitschrift "kettenwirk-praxis 1/94", 1994, Seiten 73 bis 76 "Raschelgewirkte Abstandsgewirke".

Weiterhin bevorzugt werden als Material ein Neoprenschaum, ein Polyurethanschaum oder ein Polyesterschaum verwendet, die zur besseren Belüftung bevorzugt perforiert sind.

In einer weiteren bevorzugten Ausführungsform der Bandage weist das Material eine Elastizität von 30 bis 150 % in der X-Achse und von 5 bis 70 % in der Y-Achse auf, wobei die X-Achse im angelegten Zustand zirkulär um den Unterschenkel beziehungsweise um den Fuß verläuft und die Y-Achse ebenfalls im angelegten Zustand im Unterschenkelteil vertikal, also longitudinal, und im Fußteil in Fußlängsachse verläuft.

In einer weiteren bevorzugten Ausführungsform der Bandage weist das Material für das Verbindungsteil eine Elastizität von 50 bis 200 % in der X-Achse und von 0 bis 50 % in der Y-Achse auf, wobei die X-Achse im angelegten Zustand zirkulär beziehungsweise horizontal um den Unterschenkel beziehungsweise um den Fuß verläuft und die Y-Achse ebenfalls im angelegten Zustand im Unterschenkelteil vertikal, also longitudinal, und im Fußteil in Fußlängsachse verläuft.

Schließlich stellt es eine hervorragende Ausgestaltung der Bandage dar, wenn das Unterschenkelteil oder die Zügel am Unterschenkelteil und/oder die Zügel des Fußteils Klettverschlüsse oder Druckknöpfe aufweisen.

Die zirkuläre Umfassung im distalen Unterschenkelbereich führt zu einer individuell einstellbaren Kompressionswirkung und folgt dem Verlauf der Ligamenten Tibiofibulare anterior und posterior, welche die Tibia und Fibula bandartig zusammen hält und diesen somit unterstützt.
Die Umfassung des Vorfußes, gebildet jeweils aus einem medialen und lateralen Zügel vom plantaren Bereich kommend und sich überkreuzend auf dem Fußrücken zum distalen Bereich des Unterschenkels verlaufend, wirkt stabilisierend auf das obere und untere Sprunggelenk, indem eine aktive Supination und Pronation des Vorfußes begrenzt wird und somit die Inversion des Fußes reduziert wird. Dies wirkt sich vorteilhaft auf die laterale fibulare-calcanei-Bandstruktur aus, da eine Eversion für diese Struktur eher schädlich ist. Da die Zügel der Vorfußumfassung individuell verstellbar sind, ist es möglich, je nach Indikation die Unterstützungskraft entsprechend einzustellen. Die Vorfußfassung folgt der Anatomie, dem sogenannten Steigbügel, der gebildet wird aus den Sehnen des Muskulus Tibialis anterior und Muskulus Peroneus longus.

Die Bandage schränkt kaum das physiologische Gangbild ein. Aufgrund der Aussparung der Fersenpartie wird die Gefahr von Druckstellen im Fersenauftrittsbereich vermieden und das Bodenauftrittsgefühl bleibt erhalten.

Die Erfindung wird anhand von fünf schematischen Zeichnungen eines Ausführungsbeispiels näher erläutert, und zwar die Ausführung für den rechten Fuß, ohne damit die Erfindung unnötig einschränken zu wollen. Die Ausführungsvariante der Bandage für den linken Fuß ist zu der ersteren spiegelsymmetrisch ausgeführt.

Es zeigen
- Figur 1: den Zuschnitt einer besonders vorteilhaften Ausführungsform der Bandage, und zwar das Unterschenkel- und das Fußteil getrennt,
- Figur 2: den Zuschnitt des bevorzugt eingesetzten Verbindungsteils,
- Figur 3: die angelegte Bandage, ohne daß die Zügel verschlossen sind,
- Figur 4: die halb angelegte Bandage, wobei Teile der Zügel verschlossen sind, und
- Figur 5: die angelegte Bandage.

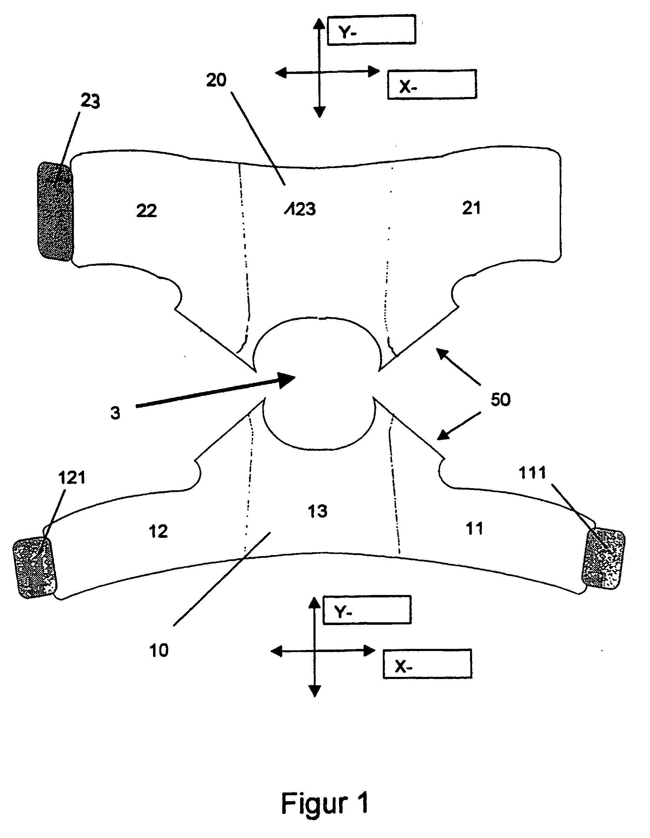

In der Figur 1 sind die Zuschnitte des Fußteils 10 und des Unterschenkelteils 20 einer hervorragend ausgestalteten Bandage 1 für das Sprunggelenk dargestellt.
Das Unterschenkelteil 20 besteht aus einem Zentralabschnitt 123, der eine rechteckige Grundform aufweist, wobei die Kante des Zentralabschnitts 123 im Fersenbereich rund ausgeschnitten ist. Am Zentralabschnitt 123 sind zwei Zügel 21, 22 angeformt. Am Zügel 22 ist des weiteren eine klettfähige Lasche 23 vorhanden, die den Verschluß des Unterschenkelteils 20 bei angelegter Bandage 1 sicherstellt.

Das Fußteil 10 weist an der medialen Seite einen ersten Zügel 11 und an der lateralen Seite einen zweiten Zügel 12 auf, die ihrerseits über einen Zentralabschnitt 13 miteinander verknüpft sind. Der Zentralabschnitt 13 ist ebenfalls im wesentlichen rechteckig geformt, lediglich die Kante des Zentralabschnitts 13 im Fersenbereich ist rund ausgeschnitten.
Sowohl am medialen Zügel 11 als auch am lateralen Zügel 12 ist jeweils eine Lasche 111, 121 befestigt, die vorzugsweise so ausgestaltet ist, daß die Zügel 11, 12 mittels eines Klettverschlusses in ihrer Position fixiert werden.

An den Zügeln 21, 22 des Unterschenkelteils 20 sowie an den Zügeln 11, 12 des Fußteils 10 sind deltaförmige Erweiterungen ausgeformt, die jeweils in einer einzigen Kante auslaufen. Jeweils zwei der Kanten werden miteinander verbunden, insbesondere vemäht, woraus zwei Nähte 50 resultieren. Die Erweiterungen sind im übrigen derart gestaltet, daß
- das Unterschenkelteil 20 und das Fußteil 10 so miteinander verbunden sind, daß bei am Fuß angelegter Bandage 1 die Fersenpartie in einer Aussparung 30 liegt, und
- das der Zentralabschnitt 123 des Unterschenkelteils 20 und der Zentralabschnitt 13 des Fußteils 10 nach dem Vernähen einen Winkel von ungefähr 90° aufweisen.

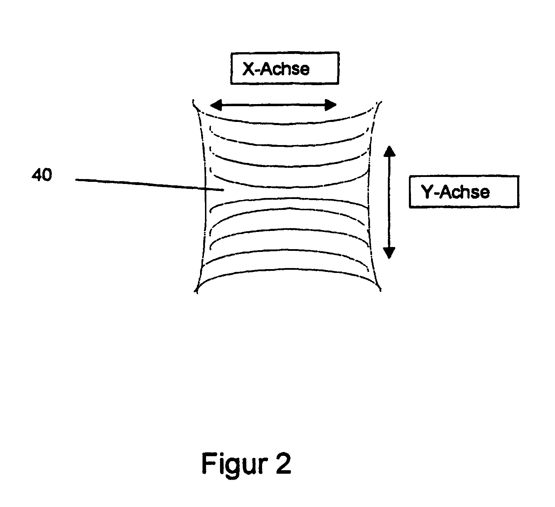

Die Figur 2 zeigt den Zuschnitt des optional eingesetzten Verbindungsteils 40. Dieses ist im wesentlichen von rechteckiger Gestalt und wird im Bereich der Zentralabschnitte 13, 123 mit dem Unterschenkelteil 20 beziehungsweise dem Fußteil verbunden, wobei auch hier insbesondere das Vemähen bevorzugt wird.

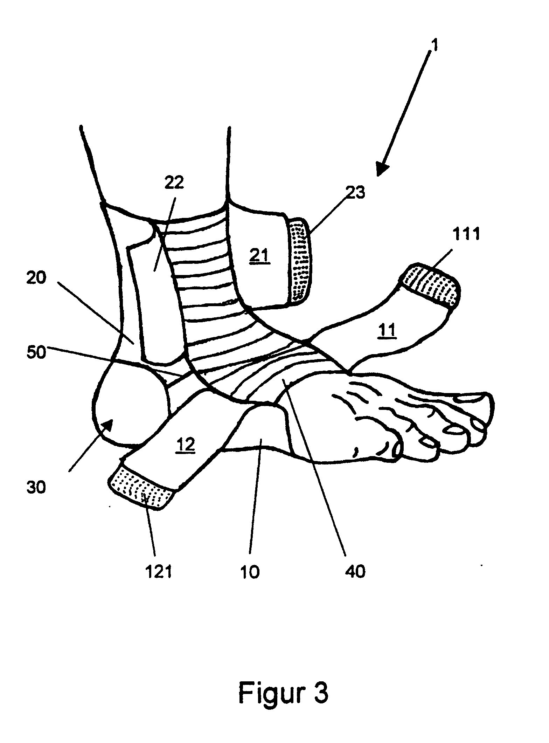

In der Figur 3 ist die angelegte Bandage1 gezeigt, ohne daß die Zügel 11, 12, 21, 22 verschlossen sind. Das Unterschenkelteil 20 umfaßt im distalen Unterschenkelbereich den Unterschenkel posterior zirkulär und ist anterior verschließbar. Das Fußteil 10 liegt unter der Fußsohle und umfaßt den Vorfuß mit dem medialen Zügel 11 und dem lateralen Zügel 12.

Das Verbindungsteil 40 umfaßt im distalen Unterschenkelbereich den Unterschenkel anterior und den Vorfuß dorsal.

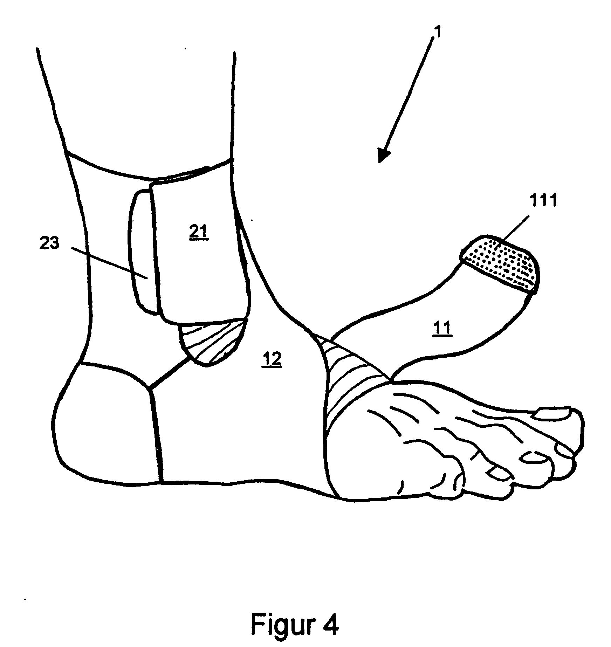

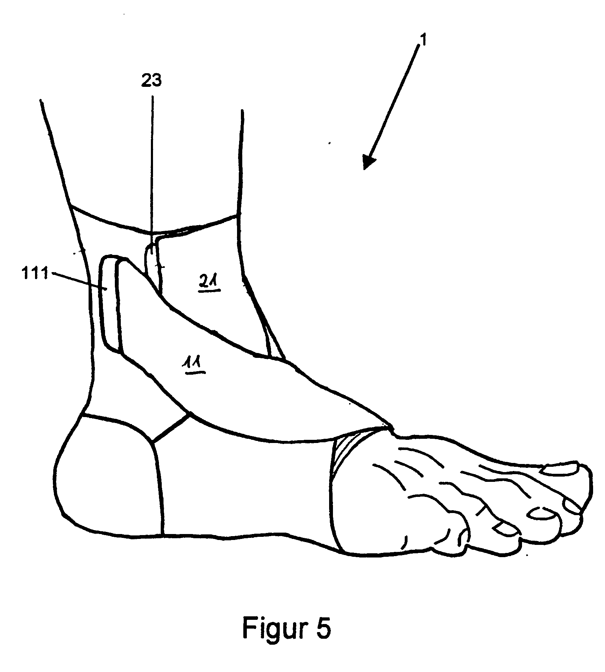

Die Figuren 4 und 5 zeigen den Vorgang des Verschließens der Bandage 1.
Die Zügel 21, 22 des Unterschenkelteils 20 werden um den Unterschenkel geführt, wobei der mediale Zügel 22 auf dem lateralen Zügel 21 durch die klettfähige Lasche 23 gehalten wird.
Anschließend wird das Fußteil 10 verschlossen. Die Zügel 11, 12 des Fußteils 10 kommen vom plantaren Bereich, kreuzen sich über dem Fußrücken und verlaufen nach medial und lateral zum distalen Bereich des Unterschenkels. Mittels der Laschen 111, 121 werden die Zügel 11, 12 auf der Bandage 1 selbst fixiert.

## Patentansprüche

1. Bandage (1) ohne starre Elemente für das Sprunggelenk mit einem Unterschenkelteil (20) und mit einem Fußteil (10) mit zwei Zügeln (11, 12), wobei
das Unterschenkelteil (20) zumindest einen Zügel (21, 22) aufweist,
das Fußteil (10) an der medialen Seite einen ersten Zügel (11) und an der lateralen Seite einen zweiten Zügel (12) aufweist,
das Unterschenkelteil (20) und das Fußteil (10) so miteinander verbunden sind, dass bei am Fuß angelegter Bandage (1) die Fersenpartie ausgespart wird,
und wobei bei angelegter Bandage (1)
das Unterschenkelteil (20) im distalen Unterschenkelbereich den Unterschenkel posterior zirkulär umfaßt und verschließbar ist, insbesondere anterior,
das Fußteil (10) unter der Fußsohle liegt und den Vorfuß mit dem medialen Zügel (11) und dem lateralen Zügel (12) umfaßt, wobei die Zügel (11, 12), vom plantaren Bereich kommend, sich kreuzend über dem Fußrücken nach medial und lateral zum distalen Bereich des Unterschenkels verlaufen,
**dadurch gekennzeichnet, dass**
ein Verbindungsteil (40) mit dem Unterschenkelteil (20) und/oder dem Fußteil (10) verbunden ist, so dass bei angelegter Bandage das Verbindungsteil (40) den Unterschenkelbereich anterior und/oder den Vorfuß dorsal umfaßt.

2. Bandage nach Anspruch 1, **dadurch gekennzeichnet, daß** das Verbindungsteil 40 mit dem Unterschenkelteil 20 und dem Fußteil 10 verbunden ist, wobei das Verbindungsteil 40 bei angelegter Bandage 1 im distalen Unterschenkelbereich den Unterschenkel anterior und den Vorfuß dorsal umfaßt.

3. Bandage nach Anspruch 1 oder 2, **dadurch gekennzeichnet, daß** das Unterschenkelteil 20 einen medialen Zügel 21 und einen lateralen Zügel 22 aufweist, die den Unterschenkel posterior zirkulär umfassen und anterior verschlossen werden.

4. Bandage nach Anspruch 1 oder 3, **dadurch gekennzeichnet, daß** das Unterschenkelteil 20 und das Fußteil 10 der Bandage 1 aus einem mindestens beidseitig kaschierten Material gefertigt sind, das ein klettfähiges Velour zur Außenseite und ein hautfreundliches textiles Gewebe zur Innenseite aufweist, oder aus einem Abstandsgewebe.

5. Bandage nach Anspruch 4, **dadurch gekennzeichnet, daß** als Material ein Neoprenschaum, ein Polyurethanschaum oder ein Polyesterschaum verwendet werden, die bevorzugt perforiert sind.

6. Bandage nach einem der Ansprüche 1 bis 5, **dadurch gekennzeichnet, daß** das Material eine Elastizität von 30 bis 150 % in der X-Achse und von 5 bis 70 % in der Y-Achse auf, wobei die X-Achse im angelegten Zustand zirkulär um den Unterschenkel beziehungsweise um den Fuß verläuft und die Y-Achse ebenfalls im angelegten Zustand im Unterschenkelteil vertikal, also longitudinal, und im Fußteil in Fußlängsachse verläuft.

7. Bandage nach einem der Ansprüche 1 bis 6, **dadurch gekennzeichnet, daß** das Material für das Verbindungsteil eine Elastizität von 50 bis 200 % in der X-Achse und von 0 bis 50 % in der Y-Achse aufweist, wobei die X-Achse im angelegten Zustand zirkulär um den Unterschenkel beziehungsweise um den Fuß verläuft und die Y-Achse ebenfalls im angelegten Zustand im Unterschenkelteil vertikal, also longitudinal, und im Fußteil in Fußlängsachse verläuft.

8. Bandage nach einem der Ansprüche 1 bis 7, **dadurch gekennzeichnet, daß** das Unterschenkelteil 20 oder die Zügel 21, 22 am Unterschenkelteil 20 und/oder die Zügel 11, 12 des Fußteils 10 Klettverschlüsse oder Druckknöpfe aufweisen.

## Claims

1. Bandage (1) without rigid elements for the ankle joint, with a lower-leg part (20) and with a foot part (10) with two straps(11, 12), the lower-leg part (20) having at least one strap (21, 22), the foot part (10) having a first strap (11) on the medial side and a second strap(12) on the lateral side, the lower-leg part (20) and the foot part (10) being connected to one another in such a way that, when the bandage (1) is applied to the foot, the heel area is left open, and, with the bandage (1) applied, the lower-leg part(20) wraps round the posterior face of the lower leg in the distal lower-leg area and can be closed, particularly on the anterior face, and the foot part (10) lies under the sole and wraps round the foot with the medial strap (11) and lateral strap (12), said straps (11, 12), starting from the plantar area, overlapping across the back of the foot and extending medially and laterally to the distal area of the lower leg **characterized in that** a connection part (40) is connected to the lower-leg part (20) and/or to the foot part (10), so that, with the bandage applied, the connection part (40) wraps round the anterior face of the lower leg area and/or wraps round the dorsal face of the foot.

2. Bandage according to Claim 1, **characterized in that** the connection part 40 is connected to the lower-leg part 20 and to the foot part 10, and, with the bandage 1 applied, the connection part 40 wraps round the anterior face of the lower leg in the distal lower-leg area and wraps round the dorsal face of the foot.

3. Bandage according to Claim 1 or 2, **characterized in that** the lower-leg part 20 has a medial strap 21 and a lateral strap 22 which wrap round the posterior face of the lower leg in a circle and are closed on the anterior face.

4. Bandage according to Claim 1 or 3, **characterized in that** the lower-leg part 20 and the foot part 10 of the bandage 1 are made of a material which is laminated at least on both sides and which has a velcro-like velour on the outside and a skin-compatible textile fabric on the inside, or of a spacer fabric.

5. Bandage according to Claim 4, **characterized in that** preferred materials are neoprene foam, polyurethane foam or polyester foam, and these are preferably perforated.

6. Bandage according to one of Claims 1 to 5, **characterized in that** the material has an elasticity of 30 to 150% in the X axis and of 5 to 70% in the Y axis, where the X axis runs in a circle round the lower leg and foot respectively in the applied state of the bandage, and the Y axis, again in the applied state of the bandage, runs vertically in the lower-leg part, i.e. longitudinally, and follows the longitudinal axis of the foot in the foot part.

7. Bandage according to one of Claims 1 to 6, **characterized in that** the material for the connection part has an elasticity of 50 to 200% in the X axis and of 0 to 50% in the Y axis, where the X axis runs in a circle round the lower leg and foot respectively in the applied state of the bandage, and the Y axis, again in the applied state of the bandage, runs vertically in the lower-leg part, i.e. longitudinally, and follows the longitudinal axis of the foot in the foot part.

8. Bandage according to one of Claims 1 to 7, **characterized in that** the lower-leg part 20 or the straps 21, 22 on the lower-leg part 20 and/or the straps 11, 12 of the foot part 10 have velcro fasteners or press studs.

## Revendications

1. Bandage (1) pour la cheville, dépourvu d'élément rigide et présentant une partie côté jambe (20) et une partie côté pied (10) dotée de deux attaches (11, 12), dans lequel la partie côté jambe (20) présente au moins une attache (21, 22), la partie côté pied (10) présentant une première attache (11) sur le côté médian et une deuxième attache (12) sur le côté latéral, la partie côté jambe (20) et la partie côté pied (10) étant reliées l'une à l'autre de façon à contourner la partie formée du talon lorsque le bandage (1) est posé sur le pied et dans lequel, lorsque le bandage (1) est posé, la partie côté jambe (20) entoure circulairement le côté postérieur de la jambe dans sa région distale et peut être fermée, en particulier sur le côté antérieur, la partie côté pied (10) se plaçant sous la plante du pied et entourant l'avant-pied au moyen de l'attache médiane (11) et de l'attache latérale (12), les attaches (11, 12) partant de la région plantaire et s'étendant en se croisant jusqu'à la région distale de la jambe en passant par le côté médian et le côté latéral de l'arrière du pied, **caractérisé en ce qu'**une partie de liaison (40) est reliée à la partie côté jambe (20) et/ou à la partie côté pied (10) de façon qu'en situation posée, la partie de liaison (40) entoure le côté antérieur de la région de la jambe et/ou le côté dorsal de l'avant-pied.

2. Bandage selon la revendication 1, **caractérisé en ce que** la partie de liaison (40) est reliée à la partie côté jambe (20) et à la partie côté pied (10), la partie de liaison (40) entourant le côté antérieur de la région distale de la jambe et le côté dorsal de l'avant-pied lorsque le bandage (1) est posé.

3. Bandage selon les revendications 1 ou 2, **caractérisé en ce que** la partie côté jambe (20) présente une attache médiane (21) et une attache latérale (22) qui entourent circulairement la jambe sur son côté postérieur et se referment sur son côté antérieur.

4. Bandage selon les revendications 1 ou 3, **caractérisé en ce que** la partie côté jambe (20) et la partie côté pied (10) du bandage (1) sont fabriquées en au moins un matériau qui est revêtu des deux côtés et qui présente, sur son côté extérieur, un velours agrippant et, sur son côté intérieur, un tissu textile à bonne affinité cutanée, ou sont fabriquées en un tissu d'écartement.

5. Bandage selon la revendication 4, **caractérisé en ce que** le matériau utilisé est une mousse de néoprène, de polyuréthane ou de polyester, de préférence perforée.

6. Bandage selon l'une des revendications 1 à 5, **caractérisé en ce que** le matériau présente une élasticité comprise entre 30 et 150 % suivant l'axe des X et entre 5 et 70 % suivant l'axe des Y, l'axe des X s'étendant en situation posée circulairement autour de la jambe ou autour du pied et l'axe des Y s'étendant, également en situation posée, verticalement et donc longitudinalement dans la partie côté jambe et suivant l'axe longitudinal du pied dans la partie côté pied.

7. Bandage selon l'une des revendications 1 à 6, **caractérisé en ce que** le matériau de la partie de liaison a une élasticité comprise entre 50 et 200 % dans l'axe des X et entre 0 et 50 % dans l'axe des Y, l'axe des X s'étendant en situation posée circulairement autour de la jambe ou autour du pied et l'axe des Y s'étendant, également en situation posée, verticalement et donc longitudinalement dans la partie côté jambe et suivant l'axe longitudinal du pied dans la partie côté pied.

8. Bandage selon l'une des revendications 1 à 7, **caractérisé en ce que** la partie côté jambe (20) ou les attaches (21), (22) de la partie côté jambe (20) et/ou les attaches (11), (12) de la partie côté pied (10) présentent des fermetures de type Velcro ou des boutons-pression.
